# EUROPÄISCHE PATENTSCHRIFT

(11) **EP 2 844 169 B1**
(45) Veröffentlichungstag und Bekanntmachung des Hinweises auf die Patenterteilung: **13.09.2017**
(21) Anmeldenummer: 13719713.3
(22) Anmeldetag: 28.03.2013
(51) Int. Cl.: A61B 17/80, A61B 17/86

(54) **KNOCHENPLATTENSYSTEM FÜR OSTEOSYNTHESE**
BONE PLATE SYSTEM FOR OSTEOSYNTHESIS
SYSTÈME À PLAQUE OSSEUSE POUR L'OSTÉOSYNTHÈSE

(30) Priorität: 03.05.2012 DE 102012103894
(43) Veröffentlichungstag der Anmeldung: 11.03.2015
(73) Patentinhaber: Merete Medical GmbH, 12247 Berlin (DE)
(72) Erfinder: CLASBRUMMEL, Bernhard, 72336 Balingen (DE); KRANZ, Curt, 14163 Berlin (DE)
(74) Vertreter: Bittner, Thomas L.
(86) Internationale Anmeldenummer: PCT/DE2013/100117
(87) Internationale Veröffentlichungsnummer: WO 2013/163985

(56) Entgegenhaltungen:
- WO-A1-2010/115403
- WO-A1-2011/076205
- US-A1- 2011 264 149

## Beschreibung

Die Erfindung betrifft Technologien auf dem Gebiet der Knochenplattensysteme für die Osteosynthese.

### Hintergrund

Knochenplattensysteme dienen dem winkelstabilen Fixieren einer Knochenplatte mittels zugeordneter Schrauben zur Osteosynthese am menschlichen oder tierischen Körper. Zahlreiche Knochenplattensysteme sind bekannt, die üblicherweise eine Knochenplatte mit einer Anordnung von mehreren Durchgangslöchern sowie zugehörige Schrauben umfassen. Hierbei können zusätzlich zu sogenannten Knochenschrauben, also Schrauben, die beim Fixieren in den Knochen eingedreht werden, Befestigungsschrauben vorgesehen sein, die ihrerseits nicht in den Knochen eingedreht werden, sondern vielmehr in ein in der Knochenplatte gebildetes Gewinde. Derartige Befestigungsschrauben dienen dann beispielsweise zur Fixierung der Knochenschrauben (vgl. zum Beispiel die Dokumente EP 1 702 577 A2, WO 2006 / 014436 A1 sowie AT 406 446 B). Auch bei dem System für eine Halswirbelsäule im Dokument DE 698 35 968 T2 wird eine Befestigungsschraube zum Fixieren mehrerer Knochenschrauben genutzt.

Im Dokument WO 2005 / 041 796 A1 ist eine Knochenplatte offenbart, die mit Langlöchern versehen ist.

Winkelstabile Platten-Schrauben-Verbindungen an Osteosyntheseplatten haben den Vorteil einer besseren Verankerung der Knochenplatte an den Knochen. Dies ist besonders bei gelenknahen Knochenbrüchen von Vorteil, da auf diese Art und Weise gelenknahe Knochenfragmente besser gefasst und fixiert werden können. Der Vorteil winkelstabiler Platten-Schrauben-Verbindungen gewinnt bei osteoporotischen gelenknahen Knochenbrüchen an Bedeutung, da Knochenschrauben ohne Winkelstabilität einen osteoporotischen Knochen schlechter fixieren können.

Winkelstabile Platten-Schrauben-Verbindungen können in monoaxiale und polyaxiale Platten-Schrauben-Verbindungen unterteilt werden.

Beispiele monoaxialer winkelstabiler Platten-Schrauben-Verbindungen sind in den Dokumenten DE 10 2005 004 841 B4 oder DE 10 2005 043 285 B3 ausgeführt. Diese Systeme sind dadurch gekennzeichnet, dass sie Schraubenköpfe mit Außengewinde aufweisen, welche in korrespondierende Innengewinde von Platten greifen. Wird eine Schraube während einer Operation eingeschraubt, bewirkt der Formschluss zwischen Außengewinde des Schraubenkopfes und Innengewinde der Knochenplatte eine winkelstabile Platten-Schrauben-Verbindung während der letzten Umdrehungen des Einschraubvorgangs.

Weiterhin wurden Knochenplattensysteme vorgeschlagen, bei denen Knochenschrauben hinsichtlich ihrer Schwenk- oder Winkelstellung in Bezug auf die Knochenplatte beim Einsatz variabel sind. Ein solches Knochenplattensystem ist beispielsweise in dem Dokument DE 10 2006 000 948 A1 beschrieben. Im Dokument WO 2007 / 025520 A1 ist eine Knochenplatte mit mindestens einer Schraube zur winkelstabilen Fixierung offenbart. Ein weiteres Beispiel einer polyaxialen winkelstabilen Platten-Schrauben-Verbindungen ist in DE 10 2005 042 766 B4 offenbart. Durch die dort beschrieben Ausbildung eines Innengewindes aus sechs Innengewindesäulen gelingt es, Kugelkopfschrauben mit einem besonderen Außengewinde in polyaxialer Richtung einzuschrauben und während der letzten Umdrehungen des Einschraubvorgangs winkelstabil zu fixieren. Aufgrund klinischer Vorteile einer polyaxialen winkelstabilen Fixierungsmöglichkeit haben sich derartige Platten-Schrauben-Systeme im klinischen Alltag immer mehr durchgesetzt.

Aus dem Dokument WO 2010 / 115403 A1 ist eine Vorrichtung zur winkelstabilen Fixation und Kompression einer Bruchstelle an einem Knochen bekannt. Die bekannte Knochenplatte verfügt über mehrere Einzellöcher, in die jeweils eine Knochenschraube einschraubbar ist. In Verbindung mit den Einzellöchern kann vorgesehen sein, dass diese über eine sichelartige Locherweiterung verfügen.

Das Dokument WO 2009 / 058969 A1 betrifft ebenfalls ein Knochenplattensystem, bei dem die Knochenplatte über mehrere Einzellöcher zur jeweiligen Aufnahme von genau einer Schraube verfügt. In den Durchgangslöchern sind Säulen mit Vorsprüngen vorgesehen, die im eingeschraubten Zustand der jeweiligen Schraube mit einem Gewinde am Schraubenkopf im Eingriff stehen. Die mehreren Säulen mit Vorsprüngen sind in dem jeweiligen Loch voneinander durch Bereiche getrennt, die frei von derartigen Vorsprüngen sind und insoweit eine glatte Oberfläche aufweisen.

Aus dem Dokument WO 2011 / 076205 A1 ist weiterhin ein Knochenplattensystem für die Osteosynthese bekannt, bei dem zum Fixieren der Knochenplatte am Knochen in ein Schwenkloch eine Schwenkschraube und in ein Klemmloch eine Klemmschraube eingeschraubt werden, derart, dass im eingeschraubten Zustand die Schwenkschraube und die Klemmschraube mehrdimensional winkelstabil fixiert sind, indem die Schraubenköpfe der Schwenkschraube und der Klemmschraube untereinander sowie mit der Knochenplatte gegen eine Relativbewegung gesichert sind, wobei die Schwenkschraube und die Klemmschraube jeweils als eine Knochenschraube ausgeführt sind.

Weiterhin offenbart Dokument DE 10 2007 005 417 A1 ein Plattenimplantat zur Anwendung an der Wirbelsäule, bei dem Knochenschrauben in ein jeweils zugeordnetes Durchgangsloch eingeschraubt werden. Der Schraubenkopf der Knochenschrauben ist mit einem Gewinde versehen, die im eingeschraubten Zustand mit Schnittlinien oder Schnittflächen in dem jeweils zugeordneten Durchgangsloch zusammenwirken.

### Zusammenfassung

Aufgabe der Erfindung ist es, verbesserte Technologien für Knochenplattensysteme zur Osteosynthese anzugeben, mit denen die mehrdimensionale Winkelstabilität der in die Knochenplatte eingeführten Schrauben optimiert ist.

Diese Aufgabe wird durch ein Knochenplattensystem zur Osteosynthese nach dem unabhängigen Anspruch 1 gelöst. Ausgestaltungen sind Gegenstand von abhängigen Unteransprüchen.

Es ist ein Knochenplattensystem zur Osteosynthese mit einer Knochenplatte, einer Knochenschraube, deren Schraubenkopf wenigstens abschnittsweise eine Oberflächenstruktur aufweist, und einer weiteren Knochenschraube vorgesehen, deren Schraubenkopf wenigstens abschnittsweise eine Oberflächenstruktur aufweist. Die Knochenplatte weist ein Durchgangsloch, welches geeignet ist, die Knochenschraube polyaxial aufzunehmen, sowie ein dem Durchgangsloch zugeordnetes weiteres Durchgangsloch auf, welches geeignet ist, die weitere Knochenschraube aufzunehmen. Das Durchgangsloch und das weitere Durchgangsloch sind über einen Durchbruch miteinander verbunden, der sich über die gesamte Dicke der Knochenplatte oder eine Teilhöhe erstreckt, so dass korrespondierende Durchgangslöcher gebildet sind.

An dem Durchgangsloch ist eine Ausbuchtung vorgesehen, welche das Durchgangsloch erweitert und dem Durchbruch gegenüberliegend angeordnet ist. Im eingeschraubten Zustand sind die Knochenschraube und die weitere Knochenschraube mehrdimensional winkelstabil fixiert, indem die Schraubenköpfe der Knochenschraube und der weiteren Knochenschraube gegen eine Relativbewegung untereinander sowie mit der Knochenplatte mittels einer gespannten Mehrpunktlagerung gesichert sind. Die gespannte Mehrpunktlagerung ist im Durchgangsloch mit Anlagerungen der Knochenschraube an Übergangsbereichen zwischen der Ausbuchtung und dem zur Ausbuchtung jeweils benachbarten Durchgangslochabschnitt gebildet. Die Übergangsbereiche sind vorzugsweise als Eckbereiche gebildet, in denen die Knochenschraube im eingeschraubten Zustand zur Anlage kommt. Die Knochenschraube ist hierdurch im Durchgangsloch an den beiden gegenüberliegenden Übergangsbereichen geklemmt.

Die Ausbuchtung des Durchgangslochs bildet eine Locherweiterung. Vorzugsweise ist das Durchgangsloch als ein Kreisloch ausgeführt. Alternativ oder ergänzend ist auch das weitere Durchgangsloch als ein Kreisloch ausgeführt.

Die Abstützung des Schraubenkopfes der Knochenschraube an den beiden Übergangsbereichen im Durchgangsloch führt zum Abstützen des Schraubenkopfes in zwei Bereichen, für die eine Verbindungslinie quer zur Verbindungsachse zwischen dem Durchgangsloch und dem weiteren Durchgangsloch liegt.

Der Schraubenkopf der Knochenschraube kann sich in die Ausbuchtung hinein erstrecken, zumindest im eingeschraubten Zustand. Vorzugsweise kommt der Schraubenkopf in der Ausbuchtung jedoch nicht zur Anlage, sondern ist beabstandet zur Oberfläche der Ausbuchtung außerhalb der Übergangsbereiche.

Die gespannte Mehrpunktlagerung kann in einer Ausgestaltung derart gebildet sein, dass unter Einbeziehung der Anlagerungen der Knochenschraube an den beiden Übergangsbereichen zwischen der Ausbuchtung und dem zur Ausbuchtung jeweils benachbarten Durchgangslochabschnitt eine Dreieckspannung hergestellt ist, die neben den beiden Übergangsbereichen die Anlagerung des Schraubkopfes der Knochenschraube an dem Schraubenkopf der weiteren Knochenschraube einbezieht. Die Dreieckspannung ist in besonderer Weise geeignet, die mehrdimensionale Winkelstabilität zu unterstützen. Eine zusätzliche Dreieckspannung kann zwischen den Anlagerungen in den beiden Übergangsbereichen und einer Anlagerung der weiteren Knochenschraube im weiteren Durchgangsloch dem Durchbruch gegenüberliegend gebildet ist.

Eine bevorzugte Weiterbildung sieht vor, dass in dem Durchgangsloch außerhalb der Ausbuchtung wenigstens abschnittsweise Radialvorsprünge und / oder -nuten gebildet sind, mit denen die Oberflächenstruktur am Schraubenkopf im eingeschraubten Zustand der Knochenschraube zumindest abschnittsweise im Eingriff stehen. Mit Hilfe der Radialvorsprünge und / oder -nuten können ein oder mehrere Gewindegänge mit Steigung gebildet sein. Das Gewinde kann vorzugsweise als Innenspitzgewinde ausgeführt sein. Die Radialvorsprünge und / oder - nuten können in einer oder mehreren Säulen angeordnet sein, die in dem Durchgangsloch gebildet sind und sich von der Ober- zur Unterseite der Knochenplatte erstrecken. In einer alternativen Ausgestaltung sind die Radialvorsprünge und / oder -nuten durchgehend vom Rand der Ausbuchtung bis zum Rand des Durchbruchs zwischen dem Durchgangsloch und dem weiteren Durchgangsloch gebildet, vorzugsweise auch in beiden Hälften des Durchgangslochs. Die Oberflächenstruktur am Schraubenkopf kann im eingeschraubten Zustand der Knochenschraube formschlüssig mit den Radialvorsprüngen und / oder -nuten im Eingriff stehen.

Bei einer Ausgestaltung kann vorgesehen sein, dass in dem weiteren Durchgangsloch wenigstens abschnittsweise Radialvorsprünge und / oder -nuten gebildet sind, mit denen die Oberflächenstruktur am Schraubenkopf im eingeschraubten Zustand der weiteren Knochenschraube zumindest abschnittsweise im Eingriff stehen. Für die bevorzugten Ausführungsformen gelten hier die oben in Verbindung mit den Radialvorsprüngen und / oder -nuten des Durchgangslochs gemachten Ausführungen entsprechend.

Eine vorteilhafte Ausführungsform sieht vor, dass die Ausbuchtung mit einer Sichelform gebildet ist. Die äußere Form der Ausbuchtung kann in einer Ausführung einem Kreisbogenabschnitt entsprechen, wobei der zugehörige Radius des Kreisbogenabschnitts kleiner als der Radius des Durchgangsloches ist. Vorzugsweise beträgt der Radius des Kreisbogenabschnitts der Ausbuchtung höchstens etwa 2/3 des Radius des Durchgangsloches. Weiter bevorzugt ist, dass der Radius des Kreisbogenabschnitts der Ausbuchtung höchstens etwa 1/2 des Radius des Durchgangsloches entspricht.

Es kann vorgesehen sein, dass die Ausbuchtung eine glatte Oberfläche aufweist. Die Ausbuchtung ist frei von Radialvorsprüngen und Radialnuten. Auch ist in der Ausbuchtung hier kein Gewinde vorgesehen.

Bei einer vorteilhaften Ausgestaltung kann vorgesehen sein, dass ein geradliniger Abstand zwischen gegenüberliegenden Enden der Ausbuchtung kleiner als der Durchmesser des Durchgangsloches ist. In einer bevorzugten Ausführungsform beträgt der geradlinige Abstand höchstens 2/3 des Durchmessers des Durchgangsloches. Weiter bevorzugt ist, dass der geradlinige Abstand zwischen den gegenüberliegenden Enden der Ausbuchtung höchstens etwa der Hälfte des Durchmessers des Durchgangsloches beträgt.

Eine Weiterbildung kann vorsehen, dass das Durchgangsloch mit einer sich zur Oberseite der Knochenplatte hin öffnenden Kugelkopfaufnahme und der Schraubenkopf der Knochenschraube als ein zugeordneter Kugelkopf gebildet sind, der im eingeschraubten Zustand der Knochenschraube wenigstens teilweise in der Kugelkopfaufnahme des Durchgangsloches angeordnet ist. Im eingeschraubten Zustand kann der Schraubenkopf der Knochenschraube, soweit er in der Kugelaufnahme aufgenommen ist, formschlüssig hierin angeordnet sein. Alternativ oder ergänzend kann das weitere Durchgangsloch mit einer sich zur Oberseite der Knochenplatte hin öffnenden Kugelkopfaufnahme gebildet sein, in welche im eingeschraubten Zustand der Schraubenkopf der Knochenschraube, welcher als ein zugeordneter Kugelkopf gebildet ist, eingreift. Insoweit kann in einer Ausgestaltung vorgesehen sein, dass sowohl die Knochenschraube als auch die weitere Knochenschraube mit einem Kugelkopf gebildet sind, der im eingeschraubten Zustand jeweils in einer Kugelkopfaufnahme angeordnet ist.

Eine bevorzugte Weiterbildung sieht vor, dass der Kugelkopf mit einem ersten und einem zweiten Gewinde versehen ist, die sich am Kugelkopf überlagernd gebildet sind. Bei dem ersten und dem zweiten Gewinde kann es sich beispielsweise um ein Rechts- und ein Linksgewinde handeln. Zusätzlich können ein weiteres Gewinde und / oder horizontal umlaufende Rillen am Kugelkopf vorgesehen sein.

In einer bevorzugten Ausgestaltung ist mit der Knochenschraube eine Schwenkschraube gebildet, und mit der weiteren Knochenschraube ist eine Klemmschraube hergestellt. Die Schwenkschraube ist im eingeschraubten Zustand in ein zugeordnetes Schwenkloch eingebracht, bei dem es sich um das Durchgangsloch handelt, welches dann verschiedene Schwenkstellungen der Knochenschraube zulässt. Die Klemmschraube ist in einem von dem weiteren Durchgangsloch gebildeten Klemmloch aufgenommen. Es kann auch vorgesehen sein, das sowohl das Durchgangsloch als auch das weitere Durchgangsloch als Schwenkloch ausgeführt sind, insbesondere dann, wenn beide Knochenschrauben mit einem Kugelkopf gebildet sind.

Ohne die nachfolgend beschriebenen Ausgestaltungen auf die Kombination von Klemm- und Schwenkschraube zu begrenzen, werden nun weitere Ausgestaltungsvarianten näher erläutert. Dies gilt in gleicher Weise für die nachfolgend beispielhaft genutzten Begriffe Klemmloch und Schwenkloch. Diese betreffen allgemein ein Durchgangsloch sowie ein weiteres Durchgangsloch.

Bevorzugt sieht eine Fortbildung vor, dass die Knochenplatte mit einem an das Klemmloch angrenzenden Dehnungsbereich gebildet ist, derart, dass die Knochenplatte beim Einschrauben der Klemmschraube in das Innengewinde des Klemmloches dem Einschraubdruck aufgrund der Wechselwirkung zwischen dem Außengewinde und dem Innengewinde zumindest teilweise nachgebend und frei von plastischer Verformung verformbar ist. Auf diese Weise können sich beim Eindrehen des Schraubenkopfes mit Außengewinde in das Innengewinde Bereiche des Klemmloches nicht plastisch, insbesondere elastisch, verformen, derart, dass sich das Klemmloch an die Form des Schraubenkopfes anpasst und die Schraube aufgrund der Umfangsspannung gegen das selbständige Herausdrehen behindert wird. Die den Dehnungsbereich vorsehende Ausgestaltung wird bevorzugt derart ausgeführt, dass das Außengewinde als konisches Außengewinde und das Innengewinde als zylindrisches Innengewinde gebildet sind.

Die Klemmschraube kann mit dem Schraubenkopf wahlweise vollständig in das Durchgangsloch eingedreht werden. Der Schraubenkopf wird hierbei durch die elastische Spannung in jeder Position einerseits drehsicher gehalten, andererseits ist aber die Knochenplatte auch nicht in ihrem plastischen Bereich irreversibel verformt, d. h. überdehnt. Auf diese Weise lässt sich erreichen, dass die Klemmschraube feinfühlig genau soweit eingedreht werden kann, wie es erforderlich ist, die Knochenplatte fest am Knochen anliegend zu fixieren und die Klemmung mit der Schwenkschraube zu erreichen, wobei gleichzeitig sichergestellt werden kann, dass der Schraubenkopf in seinem erhabensten Bereichen auch die Knochenplatte nicht überragt, sodass die fixierte Knochenplatte insgesamt - mit der Klemmschraube - eine im Wesentlichen sich an den Knochen ohne Erhebungen glatt anschmiegende Einheit bildet. Es kann in einer Ausführungsform vorgesehen sein, dass der angrenzende Dehnungsbereich mindestens teilweise als ein Steg oder Teil eines Ringes ausgebildet ist.

Bei einer Ausgestaltung kann vorgesehen sein, dass bei der Klemmschraube der Schraubenschaft eine Länge aufweist, die gleich der oder kürzer als die Länge des Schraubenschaftes der Schwenkschraube ist. In einer Ausgestaltung beträgt die Länge des Schaftes der Klemmschraube höchstens die Hälfte, bevorzugt höchstens ein Drittel der Länge des Schaftes der Schwenkschraube. Die gegenüber dem Schraubenschaft der Schwenkschraube verkürzte Länge des Schaftes der Klemmschraube unterstützt insbesondere die Vielgestaltigkeit der Schwenk- oder Winkelstellungen der Schwenkschraube bezüglich der Knochenplatte. So kann der Schaft der Schwenkschraube in einer Ausführungsform auch in einem Bereich unterhalb des unteren Schaftendes der Klemmschraube geschwenkt werden.

Eine vorteilhafte Ausführungsform sieht vor, dass bei der Klemmschraube ein Knochengewinde auf dem Schraubenschaft benachbart zum Schraubenkopf mit einem sich erweiternden Abschnitt und sich an den Gewindegrund des konischen Außengewindes im Wesentlichen anschließenden Übergangsbereich gebildet ist.

Bevorzugt sieht eine Fortbildung vor, dass in der Knochenplatte mindestens ein weiteres dem Schwenkloch oder dem Klemmloch entsprechend ausgeführtes und zum Schwenkloch und zum Klemmloch korrespondierendes Durchgangsloch gebildet ist, dass in das weitere Durchgangsloch eine weitere Knochenschraube eingeführt ist, die der Schwenkschraube oder der Klemmschraube entsprechend ausgeführt ist, und dass der Schraubenkopf der weiteren Knochenschraube im eingeschraubten Zustand mit den Schraubenköpfen, beispielsweise dem Kugelkopf und dem Schraubenkopf mit konischem Außengewinde, sowie der Knochenplatte verklemmt ist. Bei dieser Ausführungsform ist eine Anordnung von wenigstens drei einander zugeordneten Durchgangslöchern gebildet, in die eine jeweilige Knochenschraube eingedreht ist. Es können beliebige Kombinationen von Schwenkschrauben und Klemmschrauben vorgesehen sein, wobei wahlweise Gewindeabschnitte der Schraubenköpfe der beteiligten Knochenschrauben paarweise formschlüssig ineinandergreifen und sich so aufeinander abstützen und gemeinsam an der Knochenplatte gegen eine Relativbewegung gesichert sind.

Nachfolgend werden bevorzugte Ausführungsformen näher erläutert.

Das Schwenkloch und das zugeordnete Klemmloch können Teil einer sogenannten Plattenlochgruppe mit weiteren Durchgangslöchern sein oder diese bilden. Die Knochenplatte kann mehrere Plattenlochgruppen aufweisen. Zusätzliche Durchgangslöcher können als Rund- oder Langlöcher zur passgenauen Aufnahme von Rund-, Senk-, Kugel-, Linsen-, Birnen- oder Konuskopfschrauben geformt sein. Eine für die Knochenplatte besonders vorteilhafte Schwenkschraube weist einen kugelförmigen Schraubenkopf auf, der am Kopfende (Nordpol) abgeflacht ist.

Eine bevorzugte Klemmschraube ist als Senkkopfschraube mit einem zylindrischen Gewinde unterhalb des Senkkopfes ausgebildet. Die Form der Unterfläche der Senkkopfschraube kann hierbei zur Verbesserung der Kontaktfläche gerundet und passend zur Kugelform des Schwenkschraubenkopfes ausgebildet sein. Die Länge des zylindrischen Gewindes des Schraubenbolzens beträgt ein Vielfaches der Plattendicke, zum Beispiel das 0,9-fache. Hierdurch kann das zylindrische Gewinde der Klemmschraube zunächst sicher im zylindrischen Gegengewinde der Platte fassen und im weiteren Verlauf des Eindrehens die Schwenkschraube an ihren geplanten Platz drücken, so dass eine möglichst große Klemmwirkung bereitgestellt ist. Dies ist besonders bei leicht verkanteten Schwenkschrauben von Vorteil. Bei dem Vorgang des Verklemmens überragt das Zylindergewinde der Klemmschraube geringfügig die Knochenplattenunterfläche, weswegen der Beginn des schraubenspitzenseitigen Zylindergewindes als selbstschneidendes Gewinde ausgebildet sein kann, so dass das Zylindergewinde geringfügig in den zu verschraubenden Knochen eindringen kann. Der weitere in der Regel längere schraubenspitzenseitige Anteil des Bolzens der Klemmschraube ist typischerweise wie eine Knochenschraube mit einem selbstschneidenden Gewinde ausgeführt.

Das Außengewinde der Klemmschraube, welches im Gegengewinde der Knochenplatte fassen soll, kann als Konusgewinde ausgebildet sein, wobei der Neigungswinkel des Konus zur Längsachse der Schraube geringer ausfällt als der Neigungswinkel des Senkkopfes.

Die Schwenkschraube kann bei der Anwendung zuerst polyaxial eingedreht werden, was es ermöglicht, Knochenfragmente zu fixieren und an die Platte zu ziehen. Anschließend kann die Klemmschraube eingedreht werden und gleichzeitig sich selbst und die Schwenkschraube während der letzten Umdrehungen winkelstabil fixieren. Hierbei wirkt die Klemmschraube selbst als monoaxiale winkelstabile Schraube.

Die Polyaxialität der Schwenkschraube wird in einer Ausgestaltung durch den äußeren Rand des Schraubenbolzens der Schwenkschraube begrenzt, welcher in geschwenktem Zustand an den unteren Rand (entspricht der dem Schraubenkopf abgewandten Knochenplattenseite) des Schwenkloches der Platte stößt. Um eine hohe variable Einstellung der Schwenkschrauben-Längsachse zu erreichen, ist vorzugsweise der Bolzen in dem Bereich unterhalb des Schraubenkopfes von einem Gewinde befreit. Die Unterseite des Schwenkloches weist vorteilhaft ergänzend eine Fase auf, um einen Schwenkradius der Schwenkschraube zu erweitern. Hierdurch kann die Schraubenlängsachse variabel zur Lochachse bis zu einem möglichst hohen azimutalen Winkel eingebracht werden.

Um einer Zerstörung des konischen Gewindes der Klemmschraube durch Reibung an dem Kugelkopf der Schwenkschraube vorzubeugen, kann das Gewinde vorteilhaft abgerundete Spitzen aufweisen.

In einem weiteren Ausführungsbeispiel ist die Oberfläche der Schwenkschraube derart ausgebildet ist, dass die Rundkopffläche der Schwenkschraube senkrecht zum Äquator des Schraubenkopfes Längssegmente aufweist, beispielsweise zwölf Längensegmente. Jedes Längssegment ist mit einer Innenspitzgewindesäule ausgestaltet, um als formschlüssiges Widerlager für die Klemmschraube zu dienen. Somit kann eine winkelstabile formschlüssige Fixierung beider Schrauben wie folgt stattfinden. Die Klemmschraube weist ein konisches Außenspitzgewinde auf, welches einerseits eine Verklemmung mit der Platte über ein konisches Innenspitzgewinde des Klemmloches formschlüssig erlaubt. Die Schwenkschraube ist über deren Innengewindesäulen am Schraubenkopf formschlüssig fixiert. Hierbei sind die Innengewindesäulen an der Schwenkschraube zum Beispiel derart ausgebildet, dass die Oberfläche des Schraubenkopfteils der Schwenkschraube noch genügend Kugelfläche aufweist, so dass beim Festdrehen der Schwenkschraube keine Gewindezerstörung oder Gratbildung auftritt.

In einer weiteren vorteilhaften Ausgestaltung kann die Fläche des Schwenkloches mit mindestens einer Innenspitzgewindesäule ausgestattet sein, um der Montage eine höhere Winkelstabilität zu verleihen. Da die Schwenkschraube ebenfalls mit vorzugsweise zwölf Innengewindesäulen ausgestattet sein kann, können die genannten Innenspitzgewindesäulen auf der Fläche des Schwenkloches vorteilhaft mit einer angepassten oder passfähigen Gewindeform ausgebildet werden.

Mithilfe derartiger auf Flächen von Schwenklöchern ausgebildeter Innengewindesäulen wird eine erhöhte Winkelstabilität erzielt. Dies kann bei Knochenplattensystemen zur Versorgung von beispielsweise Oberschenkelbrüchen vorteilhaft sein. Ein offensichtlicher Nachteil der Innengewindesäulen in der Fläche des Schwenkloches ist eine eingeschränkte Möglichkeit, Knochenfragmente mit der Schwenkschraube heranzuziehen, was Folge des Formschlusses der Schwenkschraube mit den genannten Innengewindesäulen ist. Dieser Nachteil, also eine eingeschränkte Möglichkeit, Fragmente heranzuziehen, kann in einer Ausgestaltung klinisch gleichwohl als Vorteil genutzt werden, indem schon nach dem Einbringen einer Schwenkschraube eine bestimmte Winkelstabilität erreicht wird. Nach Einbringen einer Klemmschraube wird eine höhere und robustere Winkelstabilität der Schwenkschraube mit Hilfe der Klemmschraube erreicht.

Mittels Variation der Fläche des Schwenkloches, sei es ohne oder mit mindestens einer Innenspitzgewindesäule, kann außerdem der Grad der Winkelstabilität der Schwenkschraube vorbestimmt werden, was eine vorteilhafte Anpassung an klinische Bedürfnisse gestattet.

### Beschreibung bevorzugter Ausführungsbeispiele

Im Folgenden werden weitere Ausführungsbeispiele unter Bezugnahme auf Figuren einer Zeichnung näher erläutert. Hierbei zeigen:
Fig. 1 eine schematische Darstellung eines Teils eines Knochenplattensystems mit einem Abschnitt einer Knochenplatte sowie hierin eingeschraubten Knochenschrauben,
Fig. 2 eine schematische Darstellung eines Abschnitts einer Knochenplatte von oben,
Fig. 3 eine schematische Darstellung des Abschnitts der Knochenplatte aus Fig. 2 von schräg oben und
Fig. 4 eine schematische Darstellung einer gespannten Dreipunktlagerung für den Abschnitt der Knochenplatte aus Fig. 2.

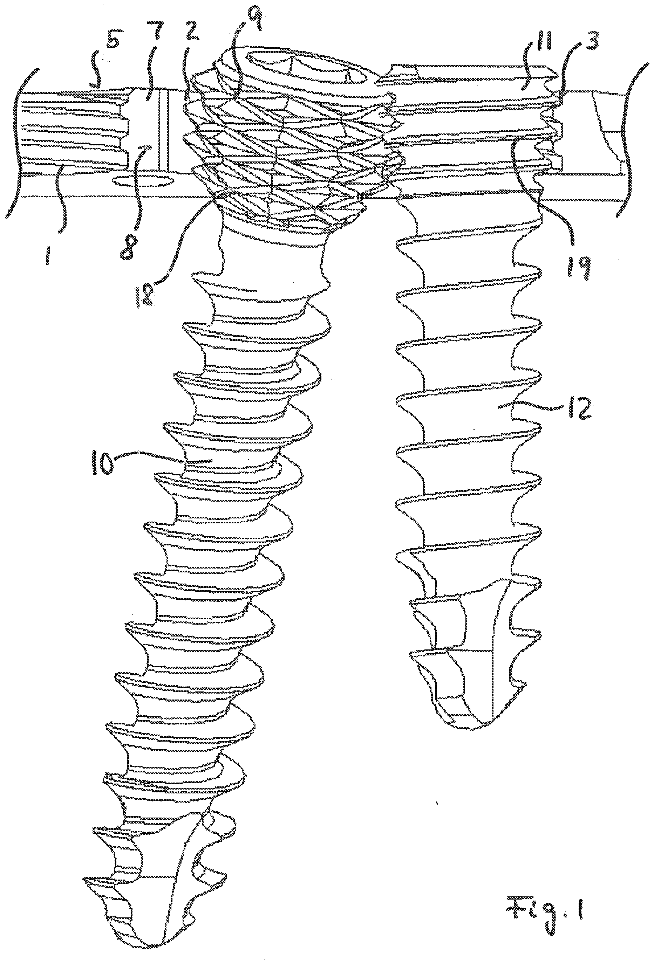

Fig. 1 eine schematische Darstellung eine schematische Darstellung eines Teils eines Knochenplattensystems mit einem Abschnitt einer Knochenplatte 1, in der ein Durchgangsloch 2 sowie ein weiteres Durchgangsloch 3 korrespondierend gebildet sind, die beim gezeigten Ausführungsbeispiel ein Schwenkloch und ein zugeordnetes Schraub- oder Klemmloch bilden. Das Durchgangsloch 2 ist mit einer Kugelkopfaufnahme 4 hergestellt, die sich zur Oberseite 5 der Knochenplatte 1 öffnet. Es kann alternativ vorgesehen sein (nicht dargestellt), dass beide Durchgangslöcher 2, 3 mit einer Kugelkopfaufnahme gebildet sind.

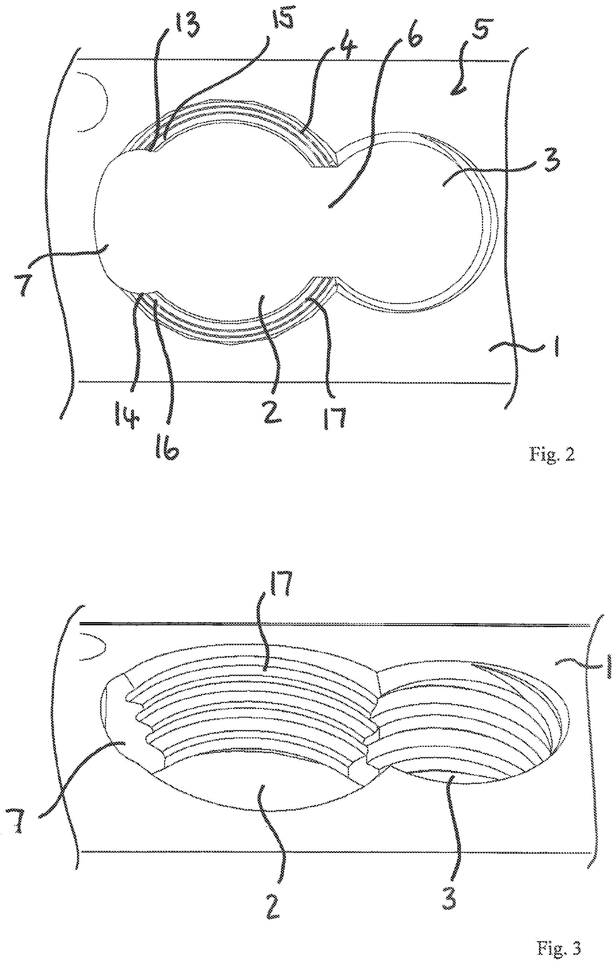

Gemäß den Fig. 2 und 3 stehen das Durchgangsloch 2 und das weitere Durchgangsloch 3 über einen Durchbruch 6 in Verbindung, so dass korrespondierende Durchgangslöcher gebildet sind. Wie am besten in Fig. 2 ersichtlich, weist das Durchgangsloch 2 gegenüber dem Durchbruch 6 eine Ausbuchtung 7 auf, die in dem dargestellten Ausführungsbeispiel eine glatte innere Oberfläche 8 aufweist.

Im eingeschraubten Zustand (vgl. Fig. 1) sind ein Schraubenkopf 9 einer Knochenschraube 10 sowie ein Schraubenkopf 11 einer weiteren Knochenschraube 12 miteinander verklemmt und hierdurch gegen eine Relativbewegung untereinander sowie zur Knochenplatte 1 gesichert. Hierbei kommt der Schraubenkopf 9 der Knochenschraube 10 in Eckbereichen 13, 14 zur Anlage, in denen die Ausbuchtung 7 an benachbarte Durchgangslochabschnitte 15, 16 grenzt, welcher ihrerseits dann durchgehend bis zum Durchbruch 6 mit Radialnuten 17 versehen sind.

Der Schraubenkopf 9 der Knochenschraube 10 verfügt über ein Außengewinde 18, welches als eine Kombination eines Rechts- und eines Linksgewindes ausgeführt ist. Das Außengewinde 18 erstreckt sich am Kugelkopf der Knochenschraube 9 umlaufend. Das Außengewinde 18 steht im eingeschraubten Zustand mit den Radialnuten 17 sowie einer Gewindekontur 19 am Schraubenkopf 11 der weiteren Knochenschraube 12 im Eingriff.

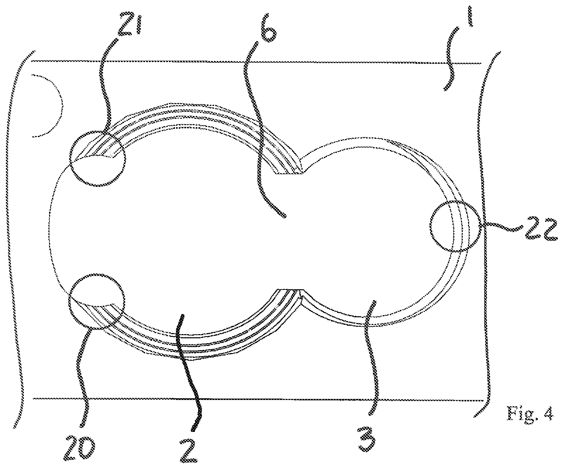

Fig. 4 zeigt eine schematische Darstellung einer gespannten Dreipunktlagerung. Kreise 20, 21 und 22 symbolisieren die Anlagerung des Schraubenkopfes 9 der Knochenschraube 10 in den Eckbereichen 14, 15 im Durchgangsloch 2 einerseits und einer Anlagerung des Schraubenkopfes 11 der weiteren Knochenschraube 12 im weiteren Durchgangsloch 3 dem Durchbruch 6 gegenüberliegend andererseits. Eine weitere Dreieckspannung ergibt sich zwischen den Anlagerungen in den Eckbereichen einerseits und dem Kontakt zwischen den Schraubenköpfen 9, 11 der Knochenschraube 10 und der weiteren Knochenschraube 12 (nicht dargestellt in Fig. 4).

## Patentansprüche

1. Knochenplattensystem zur Osteosynthese, mit:
- einer Knochenschraube (10), deren Schraubenkopf (9) wenigstens abschnittsweise eine Oberflächenstruktur aufweist,
- einer weiteren Knochenschraube (12), deren Schraubenkopf (11) wenigstens abschnittsweise eine Oberflächenstruktur aufweist, und
- einer Knochenplatte (1), die Knochenplatte (1) aufweisend
- ein Durchgangsloch (2), welches konfiguriert ist, die Knochenschraube (10) polyaxial aufzunehmen,
- ein dem Durchgangsloch (2) zugeordnetes weiteres Durchgangsloch (3), welches konfiguriert ist, die weitere Knochenschraube (12) aufzunehmen, und
- einen Durchbruch (6), welcher das Durchgangsloch (2) und das weitere Durchgangsloch (3) verbindet,
wobei im eingeschraubten Zustand die Knochenschraube (10) und die weitere Knochenschraube (12) mehrdimensional winkelstabil fixiert sind,
**dadurch gekennzeichnet, dass**
- die Knochenplatte (1) eine Ausbuchtung (7) aufweist, welche an dem Durchgangsloch (2) dieses erweiternd und dem Durchbruch (6) gegenüberliegend gebildet ist, und
- bei der mehrdimensionalen winkelstabilen Fixierung die Schraubenköpfe (9, 11) der Knochenschraube (10) und der weiteren Knochenschraube (12) gegen eine Relativbewegung untereinander sowie mit der Knochenplatte (1) mittels einer gespannten Mehrpunktlagerung gesichert sind, bei der Anlagerungen (20, 21, 22) der Knochenschraube (10) an Übergangsbereichen (13, 14) zwischen der Ausbuchtung (7) und dem zur Ausbuchtung (7) jeweils benachbarten Durchgangslochabschnitt (15, 16) im Durchgangsloch (2), zwischen den Schraubenköpfen (9, 11) sowie der weiteren Knochenschraube (12) im weiteren Durchgangsloch (3) gebildet sind.

2. Knochenplattensystem nach Anspruch 1, **dadurch gekennzeichnet, dass** in dem Durchgangsloch (2) außerhalb der Ausbuchtung (7) wenigstens abschnittsweise Radialvorsprünge und / oder -nuten (17) gebildet sind, mit denen die Oberflächenstruktur am Schraubenkopf (9) im eingeschraubten Zustand der Knochenschraube (10) zumindest abschnittsweise im Eingriff stehen.

3. Knochenplattensystem nach Anspruch 1 oder 2, **dadurch gekennzeichnet, dass** in dem weiteren Durchgangsloch (3) wenigstens abschnittsweise Radialvorsprünge und / oder -nuten gebildet sind, mit denen die Oberflächenstruktur am Schraubenkopf (11) im eingeschraubten Zustand der weiteren Knochenschraube (12) zumindest abschnittsweise im Eingriff stehen.

4. Knochenplattensystem nach mindestens einem der vorangehenden Ansprüche, **dadurch gekennzeichnet, dass** die Ausbuchtung (7) mit einer Sichelform gebildet ist.

5. Knochenplattensystem nach mindestens einem der vorangehenden Ansprüche, **dadurch gekennzeichnet, dass** die Ausbuchtung (7) eine glatte Oberfläche aufweist.

6. Knochenplattensystem nach mindestens einem der vorangehenden Ansprüche, **dadurch gekennzeichnet, dass** ein geradliniger Abstand zwischen gegenüberliegenden Enden der Ausbuchtung (7) kleiner als der Durchmesser des Durchgangsloches (2) ist.

7. Knochenplattensystem nach mindestens einem der vorangehenden Ansprüche, **dadurch gekennzeichnet, dass** die Ausbuchtung (7) im Wesentlichen spiegelsymmetrisch gebildet ist zur Verlängerung der die Mittelpunkte des Durchgangsloches (2) und des weiteren Durchgangsloches (3) verbindenden Linie.

8. Knochenplattensystem nach mindestens einem der vorangehenden Ansprüche, **dadurch gekennzeichnet, dass** das Durchgangsloch (2) mit einer sich zur Oberseite der Knochenplatte (1) hin öffnenden Kugelkopfaufnahme (4) und der Schraubenkopf (9) der Knochenschraube (10) als ein zugeordneter Kugelkopf gebildet sind, der im eingeschraubten Zustand der Knochenschraube (10) wenigstens teilweise in der Kugelkopfaufnahme (4) des Durchgangsloches (2) angeordnet ist.

9. Knochenplattensystem nach Anspruch 8, **dadurch gekennzeichnet, dass** der Kugelkopf mit einem ersten und einem zweiten Gewinde versehen ist, die sich am Kugelkopf überlagernd gebildet sind.

## Claims

1. A bone plate system for osteosynthesis, having:
- a bone screw (10), the screw head (9) of which has a surface structure, at least in sections,
- a further bone screw (12) the screw head (11) of which has a surface structure, at least in sections, and
- a bone plate (1), said bone plate (1) having
- a through hole (2) which is configured to accommodate the bone screw (10) in a polyaxial manner,
- a further through hole (3) which is associated with the through hole (2) and which is configured to accommodate the further bone screw (12),
- an aperture (6) which connects the through hole (2) and the further through hole (3),
wherein in the screwed-in position, the bone screw (10) and the further bone screw (12) are fixed at a fixed angle in mutiple dimensions,
**characterized in that**
- the bone plate (1) is provided with a protrusion (7) which is formed opposite to the aperture (6) at the through hole (2) and extending the latter, and
- the screw heads (9, 11) of the bone screw (10) and of the further bone screw (12) are secured against a relative movement with respect to each other and with respect to the bone plate (1) by means of a tensioned multi-point mount when fixed at a fixed angle in multiple dimensions, in which seating points (20, 21, 22) for the bone screw (10) are formed in the through hole (2) at transitional regions (13, 14) between the protrusion (7) and the respective through hole section (15, 16) adjacent to the protrusion (7), between the screw heads (9, 11) as well as the further bone screw (12) in the further through hole (3).

2. The bone plate system according to claim 1, **characterized in that** radial projections and/or radial grooves (17) are formed, at least in sections, in the through hole (2) outside the protrusion (7), with which the surface structure on the screw head (9) engages, at least in sections, when the bone screw (10) is in the screwed-in position.

3. The bone plate system according to claim 1 or 2, **characterized in that** radial projections and/or radial grooves are formed, at least in sections, in the further through hole (3), with which the surface structure on the screw head (1) engages, at least in sections, when the further bone screw (12) is in the screwed-in position.

4. The bone plate system according to any one of the preceding claims, **characterized in that** the protrusion (7) is formed with a crescent shape.

5. The bone plate system according to any one of the preceding claims, **characterized in that** the protrusion (7) has a smooth surface.

6. The bone plate system according to any one of the preceding claims, **characterized in that** a rectilinear distance between opposite ends of the protrusion (7) is smaller than the diameter of the through hole (2).

7. The bone plate system according to any one of the preceding claims, **characterized in that** the protrusion (7) is essentially symmetrical about the extension of the line connecting the centre points of the through hole (2) and of the further through hole (3).

8. The bone plate system according to any one of the preceding claims, **characterized in that** the through hole (2) is formed with a spherical head retainer (4) opening towards the upper side of the bone plate (1), and the screw head (9) of the bone screw (10) is formed as an associated spherical head, which is at least partially disposed in the spherical head retainer (4) of the through hole (2) when the bone screw (10) is in the screwed-in position.

9. The bone plate system according to claim 8, **characterized in that** the spherical head is provided with a first thread and a second thread which are formed so as to be superimposed on the spherical head.

## Revendications

1. Système de plaques à os pour ostéosynthèse, comprenant :
- une vis à os (10) dont la tête de vis (9) présente une structure de surface au moins par tronçons,
- une vis à os supplémentaire (12) dont la tête de vis (11) présente une structure de surface au moins par tronçons, et
- une plaque à os (1), la plaque à os (1) présentant
- un trou traversant (2) qui est configuré pour recevoir la vis à os (10) de façon polyaxiale,
- un trou traversant supplémentaire (3) attribué au trou traversant (2) qui est configuré pour recevoir la vis à os supplémentaire (12), et
- une percée (6) qui relie le trou traversant (2) et le trou traversant supplémentaire (3),
dans lequel dans l'état vissé, la vis à os (10) et la vis à os supplémentaire (12) sont fixées stables en angle de façon pluridimensionnelle,
**caractérisé en ce que**
- la plaque à os (1) présente un bombement (7), lequel est formé sur le trou traversant (2) en élargissant celui-ci et face à la percée (6), et
- dans la fixation stable en angle de façon pluridimensionnelle, les têtes de vis (9, 11) de la vis à os (10) et de la vis à os supplémentaire (12) sont assurées contre un mouvement relatif entre elles ainsi qu'avec la plaque à os (1) au moyen d'une fixation en plusieurs points serrée, sont formées dans les fixations (20, 21, 22) de la vis à os (10) sur des zones de transition (13, 14) entre le bombement (7) et le tronçon de trou traversant (15, 16) voisin respectivement du bombement (7) dans le trou traversant (2), entre les têtes de vis (9, 11) ainsi que la vis à os supplémentaire (12) dans le trou traversant supplémentaire (3).

2. Système de plaques à os selon la revendication 1, **caractérisé en ce que** des saillies et/ou rainures radiales (17) sont formées au moins par tronçons dans le trou traversant (2) en-dehors du bombement (7), avec lesquelles la structure de surface sur la tête de vis (9) est en prise au moins par tronçons lorsque la vis à os (10) est vissée.

3. Système de plaques à os selon la revendication 1 ou 2, **caractérisé en ce que** des saillies et/ou rainures radiales sont formées au moins par tronçons dans le trou traversant supplémentaire (3), avec lesquelles la structure de surface sur la tête de vis (11) est en prise au moins par tronçons lorsque la vis à os supplémentaire (12) est vissée.

4. Système de plaques à os selon au moins l'une des revendications précédentes, **caractérisé en ce que** le bombement (7) a une forme de faucille.

5. Système de plaques à os selon au moins l'une des revendications précédentes, **caractérisé en ce que** le bombement (7) présente une surface lisse.

6. Système de plaques à os selon au moins l'une des revendications précédentes, **caractérisé en ce qu'**une distance droite entre des extrémités opposées du bombement (7) est inférieure au diamètre du trou traversant (2).

7. Système de plaques à os selon au moins l'une des revendications précédentes, **caractérisé en ce que** le bombement (7) est formé essentiellement en symétrie de miroir pour prolonger la ligne reliant les points centraux du trou traversant (2) et du trou traversant supplémentaire (3).

8. Système de plaques à os selon au moins l'une des revendications précédentes, **caractérisé en ce que** le trou traversant (2) est formé avec une réception de tête sphérique (4) ouvrant en direction du haut de la plaque à os (1) et la tête de vis (9) de la vis à os (10) est formée en tant qu'une tête sphérique correspondante qui est disposée au moins partiellement dans la réception de tête sphérique (4) du trou traversant (2) lorsque la vis à os (10) est vissée.

9. Système de plaques à os selon la revendication 8, **caractérisé en ce que** la tête de vis est dotée d'un premier et d'un second filet qui sont formés en se superposant sur la tête sphérique.
